Europäisches Patentamt

European Patent Office

Office européen des brevets

(11) Publication number: **0 191 906**

**B1**

(12) **EUROPEAN PATENT SPECIFICATION**

(45) Date of publication of patent specification: **23.01.91**

(21) Application number: **85113984.0**

(22) Date of filing: **24.09.83**

(60) Publication number of the earlier application in accordance with Art. 76 EPC: **0 107 058**

(51) Int. Cl.⁵: **G 08 B 21/00**

(54) **Apparatus for monitoring the presence of a person in a bed.**

(30) Priority: **30.09.82 US 429047**
**04.05.83 US 491355**

(43) Date of publication of application:
**27.08.86 Bulletin 86/35**

(45) Publication of the grant of the patent:
**23.01.91 Bulletin 91/04**

(84) Designated Contracting States:
**AT BE CH DE FR GB IT LI LU NL SE**

(56) References cited:
**US-A-4 179 692**
**US-A-4 228 426**
**US-A-4 264 904**
**US-A-4 295 133**

(73) Proprietor: **Bed-Check Corporation**
**P.O. Box 170**
**Tulsa Oklahoma 74103 (US)**

(72) Inventor: **Musick, Jeff L.**
**4042 East 52 Place**
**Tulsa Oklahoma 74135 (US)**
Inventor: **Blaker, David G. Jr.**
**1354 East 26 Street**
**Tulsa Oklahoma 74114 (US)**
Inventor: **Blaker, Robert D.**
**5127 South Lewis Suite 18 P.O. Box 170**
**Tulsa OK 74101 (US)**
Inventor: **Vance, Dwight A.**
**101 South Joshua**
**Broken Arrow Oklahoma 74102 (US)**

(74) Representative: **Cole, David John**
**Executive Liaison Services 54 Templemere**
**Weybridge Surrey KT13 9PB (GB)**

Courier Press, Leamington Spa, England.

## Description

This invention relates to an apparatus for monitoring the presence of a person in a bed. The apparatus is primarily intended for monitoring the presence or absence of a hospital patient from a bed, but may obviously by used for monitoring persons in environments other than hospitals.

A problem continually encountered in hospitals is that of patients leaving their beds when the patients are not in a condition such that they can safely be trusted out of bed. Many instances have been reported of elderly or infirm patients leaving hospital beds and falling, breaking bones or otherwise causing injury to themselves. The patients are sometimes disoriented and confused because of the unfamiliar hospital environment or the effects of rugs used for therapeutic purposes, and such disorientation or confusion can cause the patients to wander, the patients sometimes leaving the hospital altogether. For these reasons, it is important that hospital staff be apprised if a patient leaves a hospital bed, and several types of prior art monitoring devices for this purpose have previously been developed.

US—A—4 264 904 describes a bed mat for use in detecting the presence of a patient in a bed. This sensing mat is divided into a number of parallel compartments, each of which is provided with a pair of metal strips which are normally held apart but which contact one another when a patient rests upon the appropriate compartment. The pairs of strips are wired in parallel so that, when the patient is in the bed, a circuit connected to the strips is closed by the contact between at least one pair of strips.

US—A—4 228 426 describes a monitoring system for monitoring a patient in a hospital bed; this monitoring system has a connecting unit that is adapted for connection between a nurse call button and a communication panel in a hospital room and is attached to a movable pressure-actuated switching means for placement in a bed or chair to operate an alarm in the nurse call system for remotely signalling the absence of a patient.

US—A—4 295 133 describes apparatus for monitoring a person in a bed, the apparatus comprising:

a binary signal generator capable of producing a first signal when the person is in the bed and a second signal when the person is absent from the bed, the binary signal generator having the form of a switch in a mat capable of being placed under the patient in the bed, so that the signal produced by the switch can change from the first signal to the second signal dependent upon the weight exerted thereon by the patient;

a time delay for producing a signal after the binary signal generator has generated its second signal for a predetermined time period;

a timer capable of producing signal pulses at predetermined intervals;

the time delay being arranged to respond to the signals from both the binary signal generator and the timer,

a latching device responsive to the signal from the time delay; and

an alarm signal generator capable of generating an alarm signal.

It has been found that, when using the prior art monitoring equipment disclosed in US—A—4 295 133, the nature or character of the alarm signal to be generated is important. Accordingly, the present invention provides monitoring apparatus of the type described in US—A—4 295 133, but which incorporates an alarm control device capable of varying the nature of the alarm signal produced. The present apparatus also incorporates a latch resetting device and an automatic resetting device.

Accordingly, this invention provides apparatus for monitoring a person in a bed, this apparatus being of the type described in US—A—4 295 133 but characterized in that it further comprises an alarm control device capable of varying the nature of the alarm signal produced by the alarm signal genertor, and a latch resetting device arranged to reset the latching device and to provide for automatic resetting thereof, the latch resetting device comprising a first switch for placing the apparatus in a non-operating condition in which the alarm signal will not be generated even if the binary signal generator generates its second signal for the predetermined period, a second switch for placing the apparatus in an operating condition in which the alarm signal will be generated if the binary signal generator generates its second signal for the predetermined period, and an automatic resetting device for returning the apparatus from its non-operating condition if the second switch is not operated within a predetermined delay following the operation of the first switch.

In the monitoring apparatus of the invention, although other types of timer may be used, the timer preferably comprises a frequency divider. Such a timer is conveniently of a form which can receive alternating current from the mains or another appropriate source and divide it at an appropriate rate to provide a signal pulse at predetermined intervals, conveniently about one second. These signal pulses from the timer are passed to the time delay, which also receives the signals from the binary signal generator. By monitoring the signals from both the binary signal generator and the timer, the time delay is enabled to produce a signal after the binary signal generator has generated its second signal (indicating the absence of the person from the bed) for a predetermined time period, as measured by the number of signal pulses produced by the timer. The time delay is conveniently in the form of a digital counter and is also desirably provided with a selector switch to enable a number of different periods of delay to be selected. Since movement of the person within the bed or restlessness of the person may cause occasional short-lived second signals from the binary signal generator, it is important to be able to select an appropriate time delay for the monitoring device of the invention so that such movement or restlessness of the person being monitored does not cause spurious alarm signals.

The latching device used in the monitoring apparatus of the invention can be a flip-flop (conveniently any one of a number of commercially available forms of this device) and serves to monitor the signal produced by the time delay; when the time delay produces a signal, this signal is supplied to the set terminal of the flip-flop, thereby producing appropriate output voltages at the Q and $\overline{Q}$ terminals. The outputs from the Q and $\overline{Q}$ terminals of the flip-flop can then be used by the alarm control device to cause appropriate alarm signals to be generated by the alarm signal generator. The latching action of the flip-flop ensures that once the time delay has produced a signal, the latching device will cause an alarm signal to be generated and indefinitely until the latching device is reset.

The output of the latching device is fed to the alarm control device, which is capable of varying the nature of the alarm signal produced by the alarm signal generator. Preferably, the alarm control device selects one of a plurality of possible alarm signals, the possible alarm signals desirably including any one or more of a single voltage pulse of predetermined duration, a steady predetermined voltage and a series of spaced pulses of predetermined voltage. Such selection of the appropriate form of alarm signal by the alarm signal generator ensures compatibility of the monitoring apparatus of the invention with a variety of nurse call or similar systems. Conveniently, the alarm signal generator comprises a relay which receives a signal from the alarm control sevice, the relay serving to provide a predetermined voltage on a pair of conductors which extend from the room of the person being monitored to a nursing of similar station so that an audible, visual or other type of alarm can be generated to call the attention of the nurses or other attendants. In many cases, it may be desirable to provide a separate alarm, such as an audible oscillator signal or a lamp on the monitoring apparatus itself so that functioning of the latching device will be immediately apparent to nursing or similar personnel in the room of the person being monitored.

As already mentioned, the monitoring apparatus includes a latch resetting device comprising a first switch for placing the apparatus in a non-operating condition in which the alarm signal will not be generated even if the binary signal generator generates its second signal for the predetermined period, a second switch (which may comprise a switch on the bed of the person being monitored which can be pressed by an attendant to reset the latching device) for placing the apparatus in an operating condition in which the alarm signal will be generated if the binary signal generator generates its second signal for the predetermined period, and an automatic resetting device for returning the apparatus from its non-operating condition if the second switch is not operated within a predetermined delay following the operation of the first switch.

A preferred embodiment of the invention will now be described, though by way of illustration only, with reference to the accompanying drawings, in which:

Fig. 1 is top plan view of a preferred binary signal generator (hereinafter also referred to as a "switching device");

Fig. 2 is an enlarged top plan view of part of the switching device shown in Fig. 1 with various parts broken away to show the internal construction of the switching device;

Fig. 3 is a cross section taken across the width of the device shown in Figs. 1 and 2 (i.e. vertically in Fig. 1); and

Fig. 4 is a circuit diagram of a preferred monitoring apparatus of the invention.

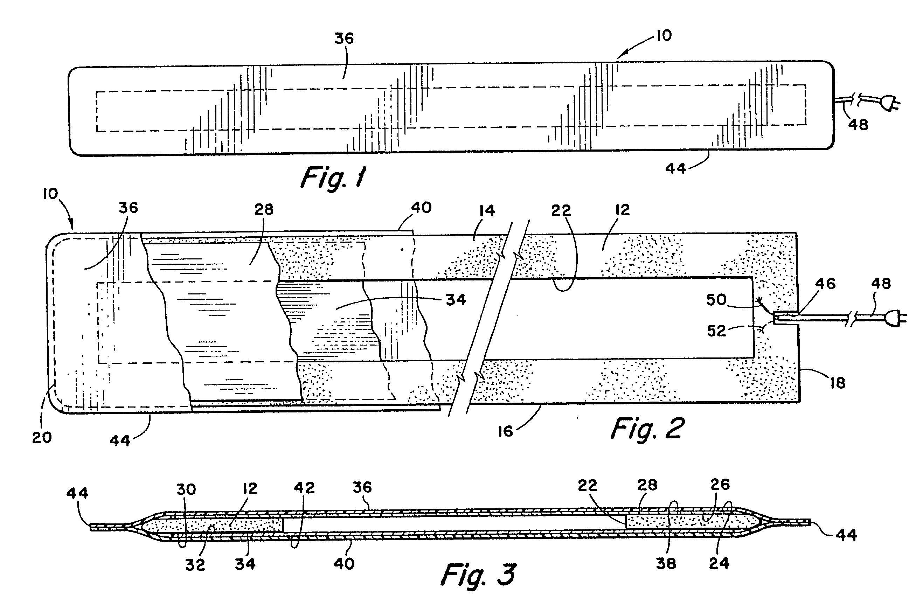

As shown in Figs. 1—3, the preferred switching device (generally designated 10) comprises a thin base member 12 which is formed of insulating flexible material, conveniently high-density polyethylene foam. The base member 12 is generally rectangular having opposed side edges 14 and 16 and ends 18 and 20. A rectangular aperture 22 is cut centrally through the base member 12, the dimensions of the aperture 22 being approximately 38 to 64 mm smaller in both width and length than those of the base member 12 itself. The base member shown in Figs. 1—3 has a length from end 18 to end 20 of aproximately 63.5 to 101.6 cm, while its width between the side edges 14 and 16 is 9.53 to 12.7 cm. The thickness of the base member measured between its surface 24 and its lower surface 30 is approximately 1.6 mm. The aperture 22 is conveniently 45 to 57 mm wide and 57.1 to 96.5 cm long, though obviously the dimensions of both the base member and the aperture can be varied quite widely.

As best seen in Figs. 2 and 3, the base member 12 is sandwiched between two thin, electrically conductive members 28 and 34, which are both slightly smaller in both width and length than the base member 12 itself. The lower surface 26 of the conductive member 28 is adhesively affixed to the upper surface 24 of the base member 12, while the upper surface 32 of the conductive member 34 is adhesively affixed to the lower surface 30 of the base member 12. In the preferred switching device shown in Figs. 1—3, the conductive members 28 and 34 are made of thin metal, such as tin foil, aluminum foil or a similar metal surface, but alternatively the conductive members may be made of metal folds bonded to a plastic backing sheet or may be formed of metalized plastic, produced for example by applying electroconductive ink to a plastic foil.

The base member 12 and the conductive members 28 and 24 are all sandwiched between two plastic cover members 36 and 40. As shown in Figs. 2 and 3, the upper cover member 36 has its lower surface bonded to the upper surface 38 of

the conductive member 28, while the upper surface of the lower cover member 40 is bonded to the lower surface 42 of the conductive member 34. The length and width of the cover members 36 and 40 are both slightly greater than the corresponding dimensions of the base member 12 so that the peripheral portions 44 of the cover members 36 and 40 extend outwardly beyond the periphery of the conductive members 28 and 34 and the base member 12. These peripheral portions 44 of the two cover members are sealed to one another so that no portions of the base member 12 of the conductive members 28 and 34 are exposed.

To provide external connections to the conductive members 28 and 34, a notch 46 is cut in the end 18 of the base member 12 and a cable 48 extends between the peripheral portions 44 of the cover members 36 and 40 into this notch 46. The cable 48 carries a first conductor 50 which is connected, between the cover members 36 and 40, to the conductive member 28, and a second conductor 52 which is similarly connected, between the cover members 36 and 40 to the second conductive member 34.

The switching device shown in Figs. 1—3 may be easily assembled in the following manner. The base member 12 has the notch 46 cut therein and adhesive is then applied to both the top and bottom surfaces 24 and 30 of the base member. The cable 48 is next inserted into the notch 46 and the conductors 50 and 52 exposed so as to lie adjacent the upper and lower surfaces 24 and 30 respectively of the base member 12. The conductive members 28 and 34 are then positioned on the base member, thereby engaging the connectors 50 and 52 respectively. The cover members 36 and 40 are then placed in the appropriate positions in contact with the conductive members 28 and 34 respectively, adhesive being placed between each cover member and the adjacent surface of the conductive member. The assembled switching device is then pressed together by means of a press or rollers so as to bond the various members rogether and the peripheral portions 44 of the cover members 36 and 40 are sealed to each other to complete the assembly of the switching device. It will be seen that all the elements used in the switching device are inexpensive and that the switching device can be expeditiously assembled. Thus, the switching device can be manufactured sufficiently cheaply to be considered a disposable item and its use limited to a single patient, although if desired the device can be sterilized for reuse.

As will be apparent to those skilled in this field, the switching device shown in Figs. 1—3 operates as follows. When the base member is in its undeformed state, as shown in Fig. 3, the flexible base member holds the conductive members 28 and 34 spaced from one another by a distance substantially equal to the thickness of the base member. Thus, in this condition, there is no electrical contact between the conductors 50 and 52. However, when a person lies, sits or otherwise disposes himself upon the switching device, the weight of the person distorts the switching device sufficiently to cause the conductive members 28 and 34 to come into contact with one another via the aperture 22 in the base member 12 at one or more places, thus providing electrical contact between the conductors 50 and 52.

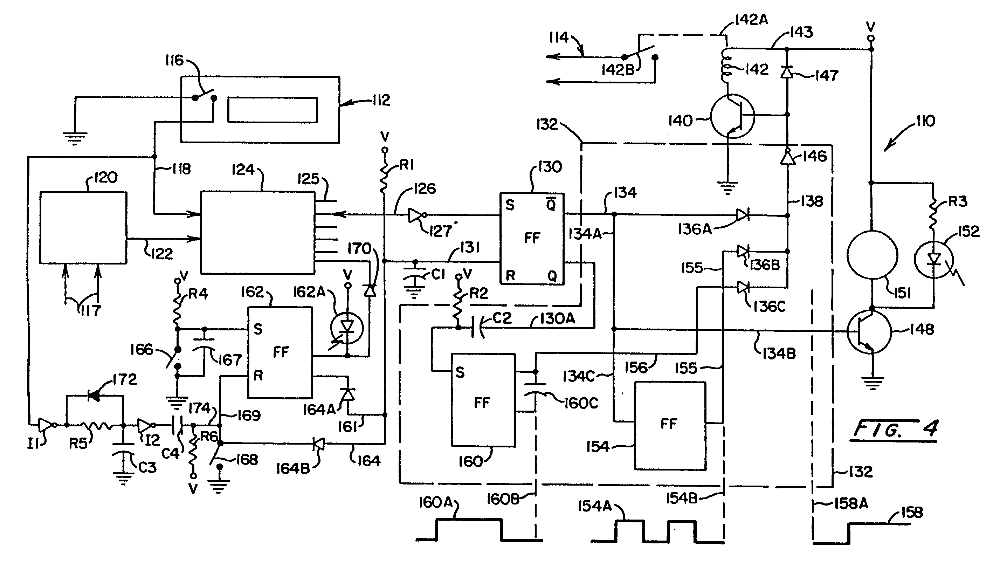

Fig. 4 shows in detail the circuit diagram of a monitoring apparatus (generally designated 110) of the invention, the associated switching device 112 being shown only schematically. This switching device 112 may be a switching device as shown in Figs. 1—3 or may be any other appropriate type of binary signal generator capable of producing a first signal when the person is in the bed and a second signal when a person is off the sensing device. Thus, as indicated schematically in Fig. 4, the switching device 112 functions as a biased-open switch, which is closed by a person resting upon the switching device.

One terminal of the switch 116 is grounded, while the other terminal is connected via a line 118 to one input of a time delay 124, which is of the binary counter type. Mains alternating current is supplied via leads 117 to frequency divider 120 which effects appropriate frequency division of the alternating input on the leads 117 and produces output pulses at a rate of one per second on a timer output line 122, which is connected to a second input of the time delay 24. The time delay 124 is arranged so that, when the switch 116 is open (thereby indicating that the person is no longer lying on the switching device) the time delay 124 begins to count the number of pulses received from the frequency divider 120.

The time delay 124 is provided with a selector switch (shown schematically as 125) which permits an operator to select a delay period of a predetermined number of seconds (or other interval depending upon the frequency of pulses chosen as the output from the frequency divider 120). The delay selected by the selector switch 125 should be chosen so as to avoid sounding an alarm when the switch 116 is only momentarily open. For example, a restless patient may momentarily lift him or herself up from the bed, thus opening the switch 116 but it is obviously not desirable to sound an alarm for each such momentary opening of the switch 116. Thus, the time delay 124 is used so that an alarm will only be sounded when the switch 116 has been semicontinously for a predetermined period selected by the selector switch 125.

Accordingly, if the switch 116 remains open for longer than the delay period predetermined by the setting of the selector switch 125, the time delay 124 produces an appropriate voltage signal on its output line 126, which extends via an inverter 127 to the S terminal of a flip-flop 130. (The inverter 127 is needed to provide the proper polarity at the S terminal of the flip-flop 130). The flip-flop 130 serves as a latching device to ensure that, once the time delay 124 has produced an output signal on the line 126, the monitoring device will generate an alarm signal for a sus-

tained period even though the time delay 124 thereafter ceases to produce its output signal.

The R terminal of the flip-flop 130 is connected via a line 131 and a resistor R1 to a terminal designated V, which is connected to a power supply. This power supply (not shown) can be of any conventional type used to supply D.C. voltage for electronic circuits and, as those skilled in the art are well aware, the appropriate voltage will be determined by the particular electronic components used. The line 131 is also connected to ground via a variable capacitor C1. As explained in more detail below, the line 131 is used to supply a reset signal for resetting the flip-flop 130.

When the flip-flop 130 is energized by receipt of a signal from the time delay 124 on the line 126, appropriate signals are generated at the Q and $\overline{Q}$ terminals of the flip-flop 130. These signals from the Q and $\overline{Q}$ terminals are supplied to a group of components, enclosed within the broken boundary 132 in Fig. 4, this group of components comprising an alarm control means. More specifically, the output from the Q terminal of flip-flop 130 is supplied via a line 130A and a variable capacitor C2 to the S terminal of a monostable flip-flop 160. Between the capacitor C2 and the S terminal of flip-flop 160, the line 130A is connected via a resistor R2 to a terminal V, which is connected to the aforementioned power supply. The output terminals of monostable flip-flop 160 are bridged by a variable capacity 160C. As indicated by the broken line 160B in Fig. 4, when the monostable flip-flop 160 receives a signal from the flip-flop 130, it generates a signal 160A which comprises a signal square voltage pulse of predetermined length, the length of the pulse being determined by the setting of the capacitors 160C. This signal is supplied along a line 156 through a diode 136C to an output line 138.

The output from the $\overline{Q}$ terminal of flip-flop 130 is supplied by a line 134 through a diode 136A to the aforementioned output line 138. As shown by the broken lines 158A extending from a line 134B (which also receives the output from the $\overline{Q}$ terminal of the flip-flop 130), the signal passing through diode 136A is a continuous voltage. The signal from the $\overline{Q}$ terminal is also supplied from line 134 via lines 134A and 134C to an astable flip-flop 154. This astable flip-flop 154 generates its output signal on a line 155; as indicated by the broken line 154B in Fig. 4, the output signal on line 155 is a series of square voltage pulses of predetermined length, this series of pulses continuing as long as the astable flip-flop 154 receives a signal from the $\overline{Q}$ terminal of flip-flop 130. The output signal on line 155 is supplied via a diode 136B to the aforementioned output line 138.

Although the output line 138 is shown in Fig. 4 as receiving all three signals passing via the diodes 136A, 136B and 136B, obviously in practice only one output signal is desired on the output line 138 at any time. Selection of the desired one of the three possible signals is effected either by removing two of the diodes 136A, 136B and 136C or, preferably, by selecting the desired signal by means of a selector switch (not shown) which connects the output line 138 to the desired one of the three diodes. It will be apparent that, when the flip-flop 130 is activated by a signal from the time delay 124, if the output line 138 is set to receive the signal via diode 136A, a continous alarm signal will exist on line 138; if the output line receives the signal via diode 136B, a series of pulses will be passed along the output line 138; and if the output line 138 is connected to the signal passing via diode 136C, a single pulse will be placed on the output line 138.

The signal on the output line 138 passes through an inverter 146. The output from this inverter 146 is supplied to the base of a NPN transistor 140. The emitter of this transistor 140 is grounded, while the collector is connected via the solenoid of a relay 142 and a line 143 to a terminal T connected to the aforementioned power supply. A diode 147 is connected between the base of the transistor 140 and the line 143.

As indicated by the broken line 142A in Fig. 4, the moveable terminal of the relay 142 is designated 142B and is connected between a pair of leads 114 which extend from the monitoring device to a nurses' station or similar location at which personnel are present so that when the relay 142 is energized appropriate signals will be provided at the nurses' station or similar location. Thus, the relay 142 acts as an alarm signal generator, producing alarm signals similar in type to those of the selected input to the line 138.

In order that the monitoring device will provide not only a remote alarm via the lines 114 but also a local alarm which can be seen and heard by personnel in the same room as the person being monitored, the monitoring device is provided with local audible and visual alarms. For this purpose, the $\overline{Q}$ terminal of flip-flop 130 is connected via lines 134, 134A and 134B to the base of a transistor 148. The emitter of this transistor is grounded, while the collector is connected via a sonic alarm 151 and a visual alarm (shown as a light emitting diode 152), and a resistor R3 arranged in parallel to the sonic alarm 151, to the same terminal V as the coil of the relay 142. It will be apparent that when the flip-flop 130 is energized and a steady signal is present at the $\overline{Q}$ terminal thereof, transistor 148 will become conducting, thereby sounding the sonic alarm 151 and illuminating the diode 152 to provide local audible and visual alarm signals to personnel in the room of the person being monitored.

As already mentioned, resetting of the monitoring device after an alarm has been sounded is effected at the R terminal of flip-flop 130. For this purpose, this T terminal is connected via lines 131 and 161 and a diode 164A to the Q output of a hold flip-flop 162. The line 131 is also connected via a line 164 and a diode 164B to one terminal of a manually-operable reset switch 168, the other terminal of the switch being grounded. The aforementioned one terminal of the switch 168 is also connected via a line 169 to the R terminal of the flip-flop 162.

The $\overline{Q}$ terminal of flip-flop 162 is connected via a light emitting diode 162A to a terminal V connected to the aforementioned power supply. The $\overline{Q}$ terminal of 162 is also connected via a diode 170 to a hold input terminal of the time delay 124. Finally, the S terminal of flip-flop 162 is connected via a resistor R4 to a terminal V connected to the aforementioned power supply, the S terminal also being connected via a normally-open switch 166 and a variable capacitor 167, arranged in parallel, to ground.

The R terminal of flip-flop 162 is provided with a time delay circuit. This time delay circuit comprises an inverter I1, the input terminal of which is connected to line 118. The output from inverter I1 is passed through a resistor R5 (this resistor being bridged by a diode 172) to the input of a second inverter I2; the line connecting the resistor R5 and the diode 172 to the input of inverter I2 is grounded via a variable capacitor C3. The output from inverter I2 passes via a capacitor C4 and a line 174 to the aforementioned line 169 connected to the R terminal of flip-flop 162; the line 174 is connected via a resistor R6 to a terminal V connected to the aforementioned power supply.

When placing a patient in the bed equipped with the switching device 112, the switch 166 is closed manually, thus grounding the S terminal of flip-flop 162, causing the $\overline{Q}$ terminal thereof to go low and turning on the light emitting diode 162A, thereby confirming the status of the flip-flop 162 and showing that the apparatus is ready for operation. After the person has been placed in the bed to be monitored, the reset switch 168 is closed momentarily by hand, thus resetting the flip-flop 162, turning off all the alarms and putting the monitoring device into operation to monitor the patient in the bed. If switch 168 is not reset manually, the circuit provides for automatic resetting of the flip-flop 162 after a time delay of approximately ten seconds provided by the time delay circuit, in order to reduce the possibility of false triggering because the switch 116 may open and close as the person is placed in the bed. Closing the switch 116 causes the output of inverter I1 to go positive, thus charging capacitor C3 via resistor R5. When the voltage on C3 reaches an appropriate value, the output of inverter I2 goes to ground, thereby momentarily grounding capacitor C4 and placing a momentary grounding pulse on line 174. This momentary grounding pulse conveyed to the R terminal of flip-flop 162 via line 169 resets flip-flop 162, thereby removing the whole signal previously passed through diode 170 to the hold input of time delay 124. If switch 16 is opened before the appropriate time delay has elapsed i.e. before the voltage on C3 has risen to a value sufficient to cause a grounding pulse to be applied to the R terminal of flip-flop 162, diode 172 will quickly discharge C3 so that when switch 116 closes C3 always starts charging from zero voltage and thus the reset pulse will only be applied after switch 116 has been closed for the time delay period. Obviously, switch 168 may be closed manually at any time to reset any alarm that has sounded.

## Claims

1. Apparatus (10, 110) for monitoring a person in a bed, the apparatus comprising:
   a binary signal generator (112) capable of producing a first signal when the person is in the bed and a second signal when the person is absent from the bed, the binary signal generator (112) having the form of a switch in a mat (10) capable of being placed under the patient in the bed, so that the signal produced by the switch can change from the first signal to the second signal dependent upon the weight exerted thereon by the patient;
   a time delay (124) for producing a signal after the binary signal generator (112) has generated its second signal for a predetermined time period;
   a timer (120) capable of producing signal pulses at predetermined intervals;
   the time delay (124) being arranged to respond to the signals from both the binary signal generator (112) and the timer (120),
   a latching device (130) responsive to the signal from the time delay (124); and
   an alarm signal generator (142) capable of generating an alarm signal,
   the apparatus being characterized in that it further comprises an alarm control device (132) capable of varying the nature of the alarm signal produced by the alarm signal generator (142), and a latch resetting device (162, 166, 168) arranged to rest the latching device (130) and to provide for automatic resetting thereof, the latch resetting device comprising a first switch (166) for placing the apparatus in a non-operating condition in which the alarm signal will not be generated even if the binary signal generator (112) generates its second signal for the predetermined period, a second (168) switch for placing the apparatus in an operating condition in which the alarm signal will be generated if the binary signal generator (112) generates its second signal for the predetermined period, and an automatic resetting device for returning the apparatus from its non-operating condition to its operating condition if the second switch (168) is not operated within a predetermined delay following the operation of the first switch (166).

2. Apparatus as claimed in claim 1 characterized in that the timer (120) comprises a frequency divider.

3. Apparatus as claimed in claim 1 or 2 characterized in that the mat (10) comprises:
   a thin base member (12) formed of a compressible insulating material and having a pair of surfaces (24, 30) on opposed sides thereof and an aperture (22) passing through the base member (12) and through the pair of surfaces (24, 30);
   first and second sheets (36, 40) of flexible insulating material each having one face (38, 42) covered with a thin layer (28, 34) of flexible, electrically conductive material, the first sheet (36) having its conductive material layer (28) in contact with one of the pair of surfaces (24) on the base member (12) and the second sheet (40) having its conductive material layer (34) in contact with the other of the pair of surfaces (30) on the base member (12); and

first and second electrical conductors (50, 52) attached to the conductive material layers (28, 34) on the first and second sheets (36, 40) respectively,

such that, when the base member (12) is in its undeformed state, it holds the conductive material layers (28, 34) on the first and second sheets (36, 40) spaced apart from one another but that when the mat (10) is subjected to the pressure of a person disposed thereon, the base member (12) deforms, thereby allowing the two conductive material layers (28, 34) to contact one another via the aperture (22) in the base member (12), and thus establishing a connection between the two electrical conductors (50, 52).

4. Apparatus as claimed in any one of the preceding claims characterized in that the alarm control device (132) varies the nature of the alarm signal produced by the alarm signal generator (142) by selecting one of a plurality of possible alarm signals.

5. Apparatus as claimed in claim 4 characterized in that the possible alarm signals include any one or more of a single voltage pulse of predetermined duration, a steady predetermined voltage and a series of spaced pulses of predetermined voltage.

**Patentansprüche**

1. Einrichtung (10, 110) zur Überwachung einer Person in einem Bett; die Einrichtung bestehend aus:

einem digitalen Signalgenerator (112), der dazu imstande ist, ein erstes Signal zu erzeugen, wenn sich die Person im Bett befindet und ein zweites Signal, wenn sich die Person aus dem Bett entfernt hat, wobei der digitale Signalgenerator (112) die Gestalt eines Schalters in einer Matte (10) besitzt, welche unter den Patienten im Bett plaziert werden kann, so daß sich das vom Schalter erzeugte Signal in Abhängigkeit des vom Patienten hierauf ausgeübten Gewichts vom ersten Signal zum zweiten Signal umwandeln kann,

einer Verzögerungsschaltung (124) zur Erzeugung eines Signals, nachdem der digitale Signalgenerator (112) sein zweites Signal über eine vorher bestimmte Zeitdauer ausgesendet hat;

einem Taktgenerator (120), der Signalimpulse in vorher bestimmten Intervallen erzeugen kann;

der derart eingerichteten Verzögerungsschaltung (124), daß sie sowohl auf Signale vom digitalen Signalgenerator (112) als auch vom Taktgenerator (120) anspricht;

einem Zwischenspeicher (130), der auf das von der Verzögerungsschaltung (124) erzeugte Signal hin anspricht; und

einem Alarmsignalgenerator (142), der ein Alarmsignal erzeugen kann,

wobei die Einrichtung dadurch gekennzeichnet ist, daß sie weiterhin über eine Alarmauswahlvorrichtung (132) verfügt, die die Art des vom Alarmsignalgenerator (142) erzeugten Alarmsignals verändern kann, sowie über eine Zwischenspeicher-Löschvorrichtung (162, 166, 168), die dazu bestimmt ist, den Zwischenspeicher (130) zurückzusetzen und für ein automatisches Löschen desselben zu sorgen, wobei die Zwischenspeicher-Löschvorrichtung einen ersten Schalter (166) enthält, der die Einrichtung in einen nicht-betriebsbereiten Zustand versetzt, in dem das Alarmsignal selbst dann nicht erzeugt wird, wenn der digitale Signalgenerator (112) sein zweites Signal über die vorher bestimmte Zeitdauer aussendet, einen zweiten Schalter (168), der die Einrichtung in einen betriebsbereiten Zustand versetzt, in welchem das Alarmsignal erzeugt wird, falls der digitale Signalgenerator (112) sein zweites Signal über die vorher bestimmte Zeitdauer aussendet, und eine automatische Rücksetzvorrichtung, die die Einrichtung aus ihrem nichtbetriebsbereiten Zustand in ihren betriebsbereiten Zustand zurückversetzt, falls der zweite Schalter (168) nicht innerhalb einer vorher bestimmten Verzugsdauer nachfolgend der Bedienung des ersten Schalters (166) bedient wird.

2. Einrichtung nach Anspruch 1, dadurch gekennzeichnet, daß der Taktgenerator (120) einen Frequenzteiler enthält.

3. Einrichtung nach Anspruch 1 oder 2, dadurch gekennzeichnet, daß die Matte (10) [folgendes] enthält:

einen dünnen Grundteil (12), der aus einem kompressiblen Isoliermaterial gebildet ist und auf gegenüberliegenden Seiten desselben ein Paar Oberflächen (24, 30) besitzt sowie eine Öffnung (22), die sich durch den Grundteil (12) und das Oberflächenpaar (24, 30) zieht;

erste und zweite biegsame Isolierplatten (36, 40), bei denen jeweils eine Oberfläche (38, 42) mit einer dünnen Schicht (28, 34) eines elastischen, elektrisch leitfähigen Materials bedeckt ist, wobei die Beschichtung aus leitfähigem Material (28) der ersten Platte (36) Kontakt zu dem einen der Oberflächenpaare (24) auf dem Grundteil (12) hat und die Beschichtung aus leitfähigem Material (34) der zweiten Platte (40) Kontakt zu dem anderen Oberflächenpaar (30) auf dem Grundteil (12) hat; und

erste und zweite elektrische Leiter (50, 52), die mit den Beschichtungen aus leitfähigem Material (28, 34) auf der ersten beziehungsweise zweiten Isolierplatte (36, 40) verbunden sind,

derart, daß der Grundteil (12), in seinem undeformierten Zustand, die Beschichtungen aus leitfähigem Material (28, 34) auf der ersten und zweiten Isolierplatte (36, 40) voneinder getrennt hält, aber daß sich der Grundteil (12), wenn die Matte (10) dem Druck durch eine darauf liegende Person ausgesetzt ist, deformiert, und dadurch ermöglicht, daß die zwei Beschichtungen aus leitfähigem Material (28, 34) über die öffnung (22) im Grundteil (12) in Kontakt kommen und so eine Verbindung zwischen den beiden elektrischen Leitern (50, 52) herstellen.

4. Einrichtung nach jedem beliebigen der vorhergehenden Ansprüche, dadurch gekennzeichnet, daß die Alarmwahlvorrichtung (132) die Art des durch den Alarmsignalgenerator (142) erzeugten Alarmsignals durch Auswahl eines aus einer Vielzahl möglicher Alarmsignale verändert.

5. Einrichtung nach Anspruch 4, dadurch gekennzeichnet, daß die möglichen Alarmsignale entweder jeden beliebigen oder mehrere eines einzelnen Spannungsimpulses von vorher bestimmter Dauer, einer stetigen vorher bestimmten Spannung und einer Reihe serieller Impulse von vorher bestimmter Spannung, enthalten.

**Revendications**

1. Appareil (10, 110) de surveillance d'une personne dans un lit, l'appareil comprenant:

un générateur de signaux binaires (112) capable de produire un premier signal quand la personne est dans le lit et un deuxième signal quand la personne n'est pas dans le lit, le générateur de signaux binaires (112) ayant la forme d'un commutateur dans un tapis (10) pouvant être placé sous le patient dans le lit de telle sorte que le signal produit par le commutateur change du premier au deuxième signal quand le poids du patient reposant sur le commutateur change;

un relais de temporisation (124) pour produire un signal après que le générateur de signaux binaires (112) a généré son deuxième signal pendant une période fixée;

un compteur (120) pouvant produire des impulsions de signaux à des intervalles prédéterminés;

le relais de temporisation (124) étant arrangé de façon à répondre aux signaux en provenance du générateur de signaux binaires (112) et du compteur (120)

un dispositif de verrouillage (130) répondant au signal du relais de temporisation (124); et

un générateur de signaux d'alarme (142) pouvant produire un signal d'alarme,

l'appareil étant caractérisé par le fait qu'il comprend également un dispositif de contrôle d'alarme (132) pouvant faire varier la nature du signal d'alarme produit par le générateur de signaux d'alarme (142) et un dispositif de remise à l'état initial du verrou (162, 166, 168) arrangé de façon à remettre à l'état initial le dispositif de verrouillage (130) et de permettre sa remise automatique à l'état initial, le dispositif de remise à l'état initial du verrou comprenant un premier commutateur (166) permettant de mettre l'appareil au repos, dans un état dans lequel le signal d'alarme ne sera pas généré, même si le générateur de signaux binaires (112) génère son deuxième signal pendant la période fixée, un deuxième commutateur (168) permettant de mettre l'appareil dans l'état de marche, dans lequel le signal d'alarme sera généré si le générateur de signaux binaires (112) génère son deuxième signal pendant la période fixée, et un dispositif de remise automatique à l'état initial permettant de faire passer l'appareil de son état de repos à son état de marche si le deuxième commutateur (168) n'est pas actionné à l'issue d'un délai fixé à la suite du déclenchement du premier commutateur (166).

2. Appareil tel que revendiqué dans la revendication 1, caractérisé par le fait que le compteur (120) contient un diviseur de fréquence.

3. Appareil tel que revendiqué dans la revendication 1 ou 2, caractérisé par le fait que le tapis (10) comprend:

une structure de base mince (12) formée d'un matériau d'isolation comprimable et ayant deux surfaces (24, 30) sur ses côtés opposés et une ouverture (22) passant à travers la structure de base (12) et à travers les deux surfaces (24, 30)

deux feuilles (36, 40) de matériau d'isolation souple ayant chacune une face (38, 42) couverte d'une couche mince (28, 34) de matériau souple, électriquement conducteur, la première feuille (36) ayant sa couche de matériau conducteur (28) en contact avec l'une des deux surfaces (24) sur la structure de base (12), et la deuxième feuille (40) ayant sa couche de matériau conducteur (34) en contact avec l'autre des deux surfaces (30) sur la structure de base (12); et

les deux premiers conducteurs électriques (50, 52) attachés aux couches de matériau conducteur (28, 34) sur la première feuille et la deuxième feuille (36, 40), dans cet ordre,

de telle sorte que quand la structure de base (12) est dans son état non déformé, elle maintienne les couches de matériau conducteur (28, 34) de la première et deuxième feuilles (36, 40) espacées l'une de l'autre mais que quand le tapis (10) est soumis à la pression d'une personne reposant sur le lit, la structure de base (12) se déforme, permettant ainsi aux deux couches de matériau conducteur (28, 34) d'être en contact l'une avec l'autre par le biais de l'ouverture (22) pratiquée dans la structure de base (12), établissant ainsi une connexion entre les deux conducteurs électriques (50, 52).

4. Appareil tel que revendiqué dans n'importe laquelle des revendications précédentes, caractérisé par le fait que le dispositif de contrôle d'alarme (132) fait varier la nature de signal d'alarme produit par le générateur de signaux d'alarme (142) en sélectionnant un signal parmi plusieurs signaux d'alarme possibles.

5. Appareil tel que revendiqué dans la revendication 4, caractérisé par le fait que les signaux d'alarme possibles comprennent l'une ou plusieurs des combinaisons: impulsion de tension unique d'une durée fixée, une tension fixée constante et une série d'impulsions espacées de tension fixée.

Fig. 1

Fig. 2

Fig. 3

EP 0 191 906 B1

_FIG. 4_

EP 0 191 906 B1